# EUROPEAN PATENT SPECIFICATION

(11) **EP 1 830 716 B1**
(45) Date of publication and mention of the grant of the patent: **24.03.2010**
(21) Application number: 05855686.1
(22) Date of filing: 27.12.2005
(51) Int. Cl.: A61B 17/00

(54) **DEVICE FOR CLOSING AN OPENING IN A BODY CAVITY OR LUMEN**
VORRICHTUNG ZUM SCHLIESSEN EINER ÖFFNUNG IN EINER KÖRPERHÖHLE ODER EINEM LUMEN
DISPOSITIF POUR FERMER UNE OUVERTURE DANS UNE CAVITE OU UNE LUMIERE CORPORELLE

(30) Priority: 27.12.2004 US 23055
(43) Date of publication of application: 12.09.2007
(73) Proprietor: Boston Scientific Limited, Christ Church (BB)
(72) Inventor: HOLMAN, Thomas, Princeton, MN 55371 (US); EIDENSCHINK, Tracee, J., Wayzata, MN 55391-9503 (US); WEBER, Jan, Maple Grove, MN 55311 (US)
(74) Representative: Joly, Jean-Jacques
(86) International application number: PCT/US2005/047169
(87) International publication number: WO 2006/071910

(56) References cited:
- US-A- 5 254 133
- US-A- 5 665 107
- US-A- 6 045 570
- US-A1- 2002 072 767
- US-A1- 2002 077 656

## Description

### Technical Field:

A closure device or system for medical applications is disclosed. More specifically, a closure device having applications for closing or stealing openings in a vessel or other body cavity is disclosed.

### Discussion of the Related Art:

There are a wide variety of procedures which require gaining internal access to blood vessels or other body cavities. During such procedures an incision is formed through the vessel or body cavity wall to insert medical instruments, such as balloon catheters, guide catheters or other devices for treatment. For example, certain cardiac or angioplasty procedures use an incision in a femoral artery or subclavian artery as an entry point for insertion of catheters or other treatment devices.

Following completion of a treatment procedure, the opening or incision in the blood vessel or other body cavity must be sealed or closed. Some prior sealing techniques include simply applying pressure to the opening until it seals itself sufficiently that the pressure may be released. However, this technique often requires that pressure must be consistently applied for an undesirable amount of time after the procedure. Similarly, this type of technique can require a patient's hospitalization to be extended until the treating physician is certain that the closure is complete.

Other techniques use or deploy a plug or closure which is deployed post operatively or upon completion of a treatment procedure to close or seal an incision. As disclosed in US 2002/072767, a guide wire is used to advance a hemostatic material, or plug into contact with a vascular puncture. However due to insertion and removal of various devices into the lumen of the blood vessel, the position of the vessel relative to the surface of the skin can change. Therefore, determining the exact position of the outer wall of the blood vessel or cavity can be difficult when deploying various closure devices.

Similarly, when entry is gained into the lumen of the blood vessel by puncturing the blood vessel, using for example a self-sticking needle as disclosed in U.S. Patent No. 5,425,718, the vessel may not have been punctured in a direction entirely orthogonal to the longitudinal axis of the blood vessel. Instead, the blood vessel may be punctured in a "side stick" fashion in which case the puncture is made in an off-center position. In such punctures, it is difficult to locate the outer wall of the blood vessel to deploy closure devices. The present invention addresses some or all of these and other problems and provides advantages over prior art sealing or closure techniques or systems.

### SUMMARY OF THE DISCLOSURE

A closure device or system to seal or close an opening in a body lumen or cavity is disclosed. The closure device or patch is positioned proximate to an entry site to the vessel or body cavity. A needle or piercing instrument is inserted through the patch to form an incision through the vessel or body wall to insert a treatment device or instrument. Following withdrawal of the treatment device or instrument, the patch seals or closes the incision.

Flowable media or gel may be used in conjunction with the patch to assist in sealing the incision. Various forms of closure device or patch are disclosed with or without various means for closing the opening in the patch through which the needle and treatment device passes. The opening in the patch may be preformed or formed by the needle. A method of closing an opening in a body lumen or cavity is also disclosed, but not claimed. These and other features provide advantages over the prior art.

### BRIEF DESCRIPTION OF THE DRAWING

FIG. 1 illustrates an embodiment of a closure device including a patch.
FIG. 1A illustrates an embodiment of a closure device including a patch having a pre-formed opening or slit.
FIGS. 2A-2E progressively illustrate an embodiment of a closure system including a patch disposed in a delivery sheath and a needle slidably disposed in the delivery sheath to form an incision at an entry site.
FIG. 3 illustrates an embodiment of a patch including a body layer and an adhesive layer that adheres to a vessel or body wall.
FIG. 4 illustrates a closure system including a patch suturable to a vessel or body wall to form a closure following treatment.
FIGS. 5A-5E illustrate embodiments of closure devices including heat sealable closures.
FIGS. 5F-5G illustrate embodiments of a closure device including an energizable closure.
FIGS. 6A-6D schematically illustrate embodiments of a closure device or patch including interlockable elements to form an openable/closeable access opening.
FIGS. 7A-7C schematically illustrate an embodiment of a closure device or patch formed of a plurality of strands having hook and loop interlockable elements to form an openable/closeable access opening.
FIGS. 8A-8D schematically illustrate an embodiment of a closure assembly or kit including a flowable media to close or seal an opening.
FIG. 9 schematically illustrates a closure assembly or kit including a patch and flowable media disposed in a delivery sheath to close or seal an opening.
FIG. 10 illustrates a closure system proximate a femoral artery or vessel to seal or close an opening in the femoral artery or vessel.
FIG. 11 is a flow chart illustrating one embodiment of application steps to seal or close an opening.

### DETAILED DESCRIPTION OF THE ILLUSTRATIVE EMBODIMENTS

During medical procedures, such as an angioplasty procedure, an incision or opening is made through a vessel or cavity wall to access a lumen or cavity. Catheters or other medical instruments are inserted into the body cavity or lumen, through the opening, for performing the treatment. Following treatment, the incision or opening to the body cavity or lumen must be sealed or closed. Embodiments of the present invention relate to a closure device which is deployed at an entry site, prior to incision, to seal or close the incision following treatment.

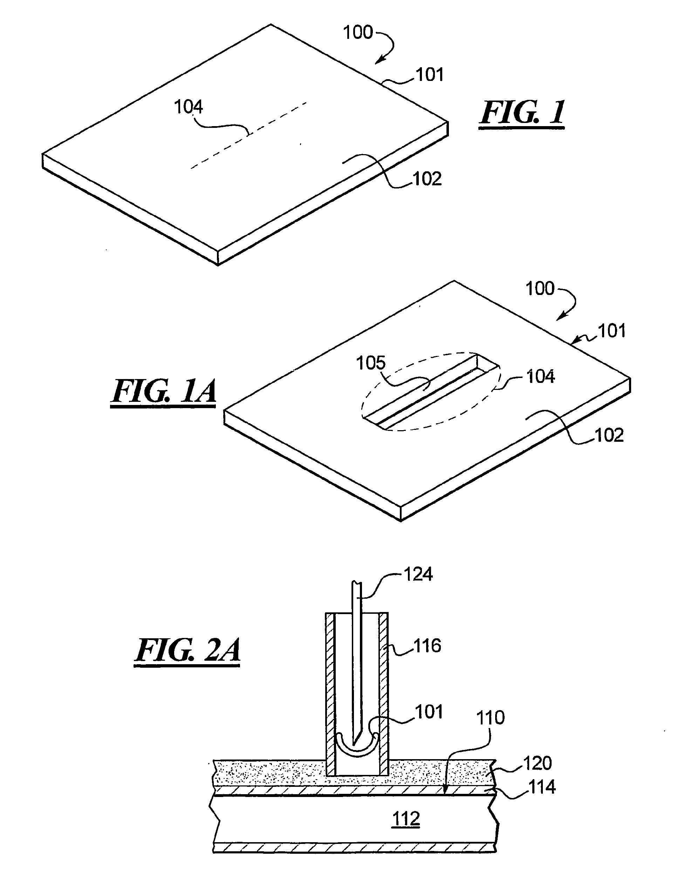

FIGS. 1 and 1A illustrates an embodiment of a closure device 100 to seal or close an incision following treatment. As shown the closure device 100 includes a patch 101 having a bioresorbable patch body 102.

In the illustrated embodiment of FIG. 1, the patch 101 is formed of a puncturable material such that a needle can be advanced to puncture or pierce the patch 101 to form an access opening 104 (shown in phantom in FIG. 1) through the patch 101 at a treatment site. As described below, after the needle punctures the patch 101, the needle is advanced to form the incision through the body vessel or cavity. Treatment devices can then be advanced through the opening 104 in patch 101 and through the incision in the vessel or body cavity. After the treatment procedure is finished, the treatment devices are withdrawn and opening 104 closes upon itself thus sealing the incision in the wall of the vessel or body cavity.

In an alternate embodiment illustrated in FIG. 1A, the patch body 102 includes a pre-formed slit or opening 105 that can be deformed or enlarged to form access opening 104 for insertion of the medical instrument or catheter. Following treatment, the pre-formed slit or opening is sealable or closable to seal or close the incision in the body vessel or cavity. Alternatively, slit 105 need not fully be pre-formed, but is simply scored or thinned or otherwise configured for ease of puncture.

The patch 101 is deployed as progressively illustrated in FIGS. 2A-2E. In the embodiment illustrated in FIGS. 2A-2E, the patch 101 is deployed adjacent a blood vessel 110, such as an artery having a lumen 112 enclosed by a vessel wall 114. Application of the patch 101, however, is not limited to a blood vessel as shown. Instead, embodiments of the closure device disclosed herein can be used to seal incisions or openings in other body cavities as well.

As shown in FIG. 2A, an opening is first made through the skin and subcutaneous tissue 120. The patch 101 is then deployed at an entry site (which will be the site of entry into lumen 112 of vessel 110) prior to creation of the incision at the entry site. The patch 101 can illustratively be deployed using a delivery sheath 116. As shown, the distal end of the delivery sheath 116 is advanced through subcutaneous tissue 120 (and can be optionally used to simultaneously create the opening in tissue 120) and the patch 101 is proximate to the vessel or cavity wall 114.

In the embodiment illustrated, the positioned patch 101 is constrained in a bent or folded configuration in the delivery sheath 116 and unfolds or expands upon deployment. The patch 101 is advanced through sheath 116 by a plunger or needle or other elongate member until it engages wall 114. Sheath 116 can be maintained in place when patch 101 is pierced, or withdrawn so the patch 101 is fully deployed prior to being pierced. In either case, the deployed patch 101 can optionally adhere to the vessel or cavity wall 114 to provide an entry and seal following treatment.

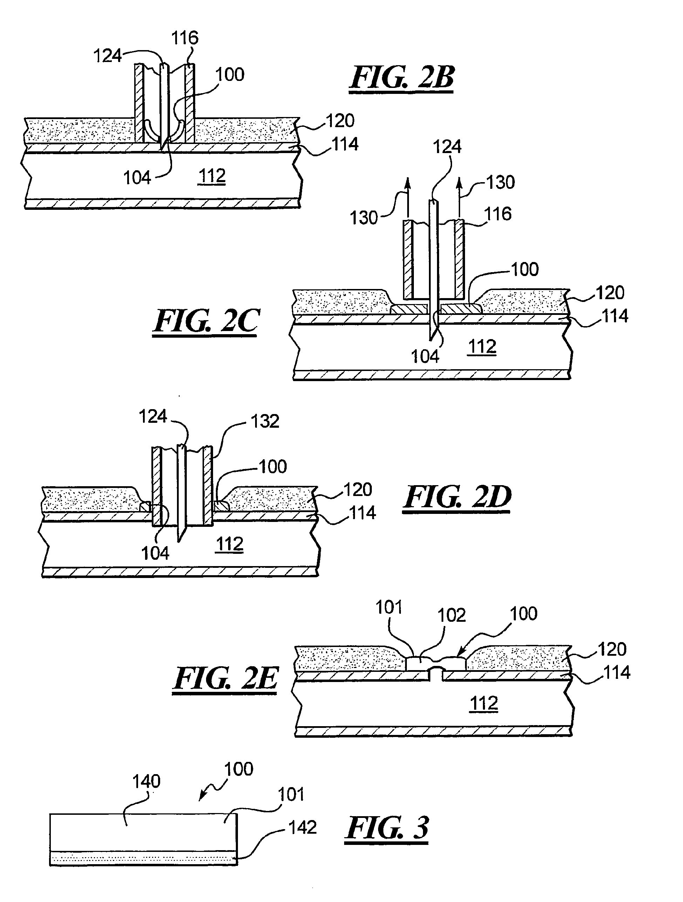

Once patch 101 is located adjacent wall 114, a needle 124 is also disposed in, and advanced distally through, the delivery sheath 116 to create an incision or opening through patch 101 and the wall 114 of the blood vessel at the site of the patch 101. For example, in the illustrated embodiment, the needle 124 is advanced through patch 101 to create an opening 104 in the patch 101 (as shown in FIG. 2B) and to form an incision in the vessel wall 114 to provide access to the vessel lumen 112 as illustrated in FIG. 2C. The treatment in lumen 112 is then performed. This can be done by inserting other catheters or treatment devices through opening 104 and the incision..

As illustrated in FIG. 2C, following deployment of the patch 101 and formation of the incision, the delivery sheath 116 can be withdrawn as illustrated by arrow 130. As shown in FIG. 2D, an introducer sheath 132 is then inserted through the incision into the body lumen 112. Catheters or instruments are then inserted into the body lumen for treatment through the introducer sheath 132. Thus, in an embodiment illustrated in FIGS. 2C-2D, the delivery sheath 116 is withdrawn and a separate introducer sheath 132 is deployed to insert catheters or instruments for treatment. Alternatively, the delivery sheath 116 (shown in FIG. 2C) can form the introducer sheath for inserting catheters or instruments into the body lumen for treatment.

Following completion of the treatment procedure, the introducer sheath 132 and the catheters or other instruments are withdrawn and the patch 101 remains in-situs to seal or close the incision as illustrated in FIG. 2E. Thus as described, the patch 101 is deployed prior to creating an opening in the vessel, to provide a seal following completion of a treatment procedure performed through the opening.

In illustrated embodiments, the access opening through the patch body 101 is self sealing upon withdrawal of the catheter or treatment instruments, although the invention is not limited to a self sealing patch, as will be described. For example, in illustrated embodiments, the patch body 102 is formed of a self sealing or gel-like material such that upon withdrawal of the catheters or instruments, the access opening fills in or closes to seal the incision in the body lumen or vessel.

In an illustrative embodiment, the patch body includes a clot forming or coagulant material which reacts with the body to seal the incision upon withdrawal of the catheters or instruments. Other self-sealing embodiments include a spring activated closure system or another type of mechanically biased closure system to close the access opening through the patch 101 upon removal of treatment devices or instruments. This can, for example, be accomplished simply by the natural bias of the patch material to return to its original position, with the opening 104 (as shown in FIG. 2E.) closed. Alternatively, this can be accomplished with other biasing techniques.

As described above, the patch body is illustratively formed of a bioresorable material that adheres to the vessel or cavity wall 114. For example, the patch can be formed of a material having sufficient surface tension to adhere to the vessel wall upon deployment. In an alternate embodiment illustrated in FIG. 3, the patch 101 includes a body layer 140 and an adhesive layer 142 such as a pressure adhesive layer to adhere to a vessel or cavity wall upon deployment. In illustrated embodiments, the body layer 140 can be formed of various materials such as a collagen material, silicone rubber, polyurethane, cellulosic polymer and poly-n-acetyl glucosamine material or alternate bioresorbable materials. The adhesive layer 142 can also be formed of any suitable adhesive.

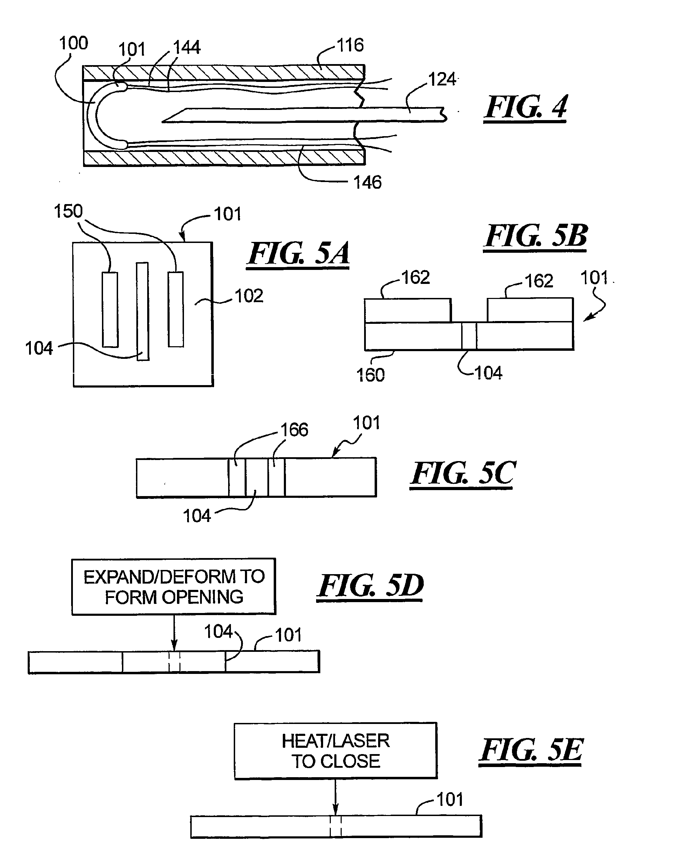

Alternatively, as illustrated in FIG. 4, the patch 101 can be secured to the vessel or cavity wall such as wall 114 shown in FIGS. 2A-2E) via suturing. As shown in FIG. 4, patch 101 includes a plurality of fibers or fiberous threads 144 which are used to suture the patch 101 to the vessel or cavity wall via a suturing needle (not shown). The patch 101 and suturing needle are disposed in delivery sheath 116 for in-situs deployment proximate to the vessel or cavity wall. Such in-situs suturing devices which are used to suture through a catheter or other sheath are well known.

In another embodiment, following treatment, the access opening through the patch can be heat sealable or closable as illustrated in FIGS. 5A-5E. In the illustrated embodiment of FIG. 5A, the patch 101 includes heating elements 150 proximate to a pre-formed or punctured access opening 104. The heating elements 150 are energized to heat seal opening 104 following withdrawal of the catheters or instruments.

In particular in the embodiment illustrated in FIG. 5B, the patch 101 includes a multiple layer structure or composition including a lower or inside heat sealable or fusable layer 160, such as soft urethane or styrene-isobutylene-styrene block copolymer (SIBS) and an outer heating element or material 162 such as an RF heating element or layer, such as a tungsten layer or material. The RF heating element or layer 162 is energized via an RF heating source to heat a portion of the heat sealable layer 160 proximate to the pre-formed or punctured opening 104-1 to seal or fuse the access opening following completion of the treatment procedure. Alternatively the access opening 104 or patch 101 can be sealed using ultrasound or other techniques. Although FIGS. 5A-5B illustrate a multiple layer heat sealable patch or structure, application is not limited to separate structural layers or bilayers for the heating element and heat sealable constituent or material.

In another embodiment the opening of the patch 101 or closure device is heat closable via energization of a shape memory activated seal or closure or closure assembly including a shape memory alloy, such as a super-elastic Nitinol (nichel titanium alloy) or a shape memory polymer.

In one embodiment illustrated in FIG. 5C, the shape memory activated closure includes shape memory alloy (SMA) plates or elements 166 which are heat activated to seal or close the access opening 104. For example in the illustrated embodiment, insertion of treatment devices or instruments applies stress or force to the SMA elements 166 to enlarge or open a pre-formed or punctured opening.

The applied force strains the SMA elements 166 or the SMA elements undergo a dimension change in response to the applied force or stress. The strain or dimension change is elastically recoverable by heating the SMA elements or plates 166. Thus, following treatment, the SMA plates or elements 166 are heated to activate the shape memory or elastic recovery so that the SMA plates or elements 166 return to the pre-stressed dimension to close or seal the enlarged or opened access opening.

Alternatively, the patch 101 can include SMA plates or elements 166 which are opened via application of heat from a heat source or a local heating element. For example in an illustrated embodiment, the SMA plate or elements 166 are heated to undergo a dimension or shape change to open or enlarge an opening to insert a medical device or instrument. Following treatment, heat is no longer supplied so that the shape memory plates or elements 166 return to a preheated profile or dimension to close the access opening following treatment.

In another embodiment illustrated in FIGS. 5D and 5E, the closure assembly includes a patch 101 formed of a shape memory polymer (SMP) or including a shape memory polymer closure element, such as a biodegradable and biocompatible shape memory polymer having a shape recovery or transition temperature in the range of 30°-70°C (or 86°-150°F).

In an illustrative embodiment as shown in FIG. 5D an opening 104 is formed in the patch 101 while the SMP or patch is at or above the transition temperature. Thereafter the patch is cooled below the transition temperature and is deployed at an entry site as previously described with reference to FIGS. 2A-2C. Medical instruments or catheters are inserted through opening 104 for treatment. Following treatment the patch 101 or SMP is heated above the transition temperature to close the opening 104 using a heating element or source such as a laser.

Alternatively, the opening can be formed in-situs and then the SMP can be heated to close or seal the opening following treatment and application is not limited to a particular embodiment shown. Thus in some illustrative embodiments, the access opening of the patch is heat activated to provide a seal or closure for the incision following treatment.

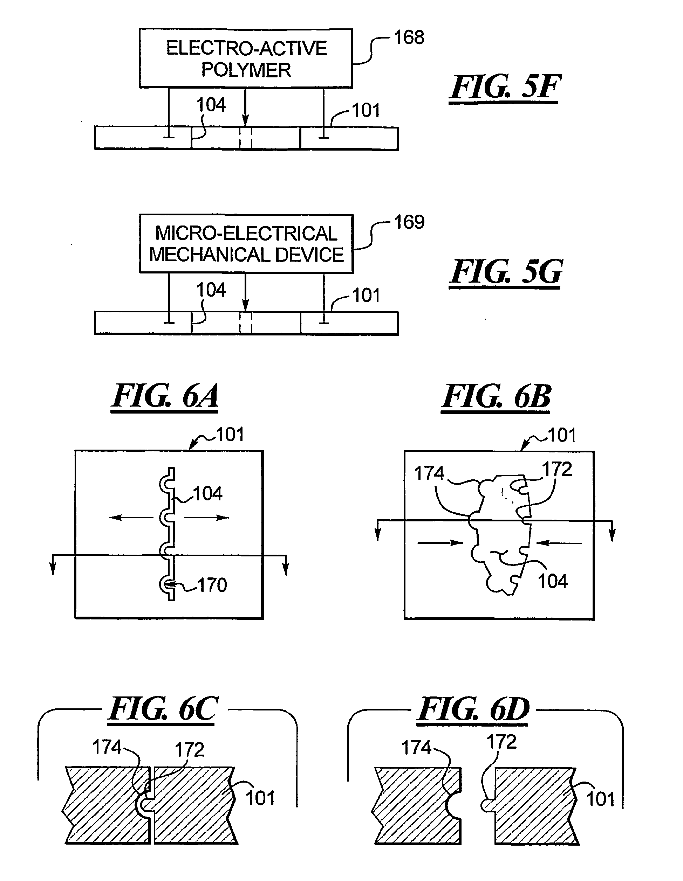

Alternatively, the closure device includes a patch 101 having an electro-active polymer closure or material 168 which is electrically activated to form an access opening 104 through the patch 101 or close an access opening as illustrated in phantom in FIG. 5F following treatment. In another embodiment, the patch 101 can include a micro-electromechanical device (MEMS) 169 which is energized to open the patch (e.g. access opening 104) for treatment or close the device (as shown in phantom) following treatment as illustrated in FIG. 5G.

The closure of the present invention is not necessarily limited to application of shape memory or electro or electromechanical closure elements. Other spring-type closures can be used to seal or close a pre-formed or punctured opening. For example, a closure can be opened against a spring bias to insert medical devices or instruments for treatment and upon removal of the bias force or medical instruments following treatment, the closure closes to seal or close the incision. Alternatively, the closure system can incorporate other energizable or heat activated closures. For example, the closure can incorporate "bucky" paper to open and close an access opening for treatment.

FIGS. 6A-6D illustrate a closure or patch including an openable/closeable slit or opening which is openable for treatment and is closeable following treatment to seal or close the incision. In the particular embodiments illustrated in FIGS. 6A-6D, the patch 101 includes an interlockable connector or connectors 170 to open and close the slit or opening 104. The interlockable connector or connectors 170 illustratively include a plurality of spaced teeth or protrusions 172 which are insertable into a plurality of receptacles or cavities 174 to form an interlockable connection.

For treatment, the interlockable connectors are opened to form an access opening 104 for insertion of treatment devices as illustrated in FIG. 6B. The interlockable connectors 170 can be opened prior to patch deployment or following deployment at the entry site simply by mechanically exerting a separating pressure (such as the surgeon applying the pressure with his or her hands).

Following treatment, the interlockable connectors 170 are closed via insertion of the protrusions or teeth 172 into the cavities or sockets 174 to close the access opening and incision, as illustrated in FIG. 6A. This is illustratively done by exerting a closing pressure (such as by squeezing the connectors 170 into the corresponding sockets). Application of the interlockable closure or connector is not limited to a plurality of protrusions 172 or cavities 174 and a single elongate protrusion or channel can be used to form an interlockable connection or connector to close or seal a body incision following treatment. As illustrated in FIGS. 6C and 6D, the interlockable connectors can have various shapes or designs to close or seal an opening or incision.

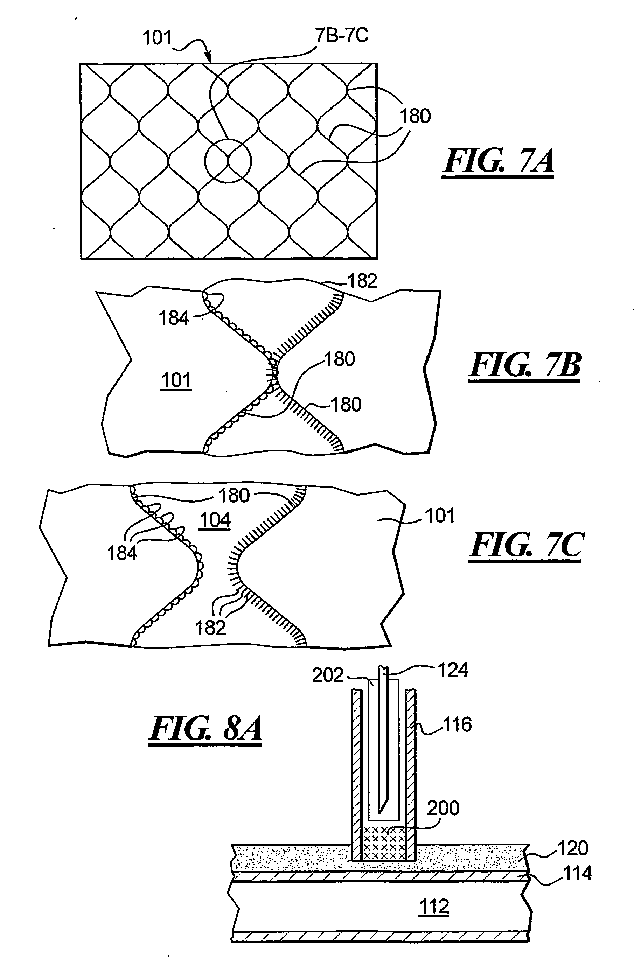

FIGS. 7A-7C illustrate another embodiment of a closure or patch 101 including an openable/closable opening 104 used to insert medical devices for treatment and closeable to seal or close an incision following treatment. As shown, the patch 101 is formed of a mesh of interlockable strands 180. The interlockable strands 180 are sinusoidal shaped and have interlockable tips to form an openable mesh-like structure. The strands 180 are interlockable via hook 182 and loop 184 connectors formed on the patch or on a portion of the patch as diagrammatically illustrated in FIGS. 7B-7C.

For treatment, force is supplied to separate the hook and loop connectors 182, 184 to form an access opening 104 to insert a treatment device or catheter as illustrated in FIG. 7C. Upon completion of treatment, force is supplied to bias the strands 180 and hook and loop connectors 182, 184 together to close the access opening formed between strands 180. For example, an operator or physician can pull hook and loop connectors 182, 184 apart to form the opening and pinch the hook and loop connectors 182, 184 closed following treatment as illustrated in FIG. 7B. The mesh-like patch can be coated with a coagulant to promote clotting to seal the incision following treatment.

The patch 101 shown in FIGS. 7A-7C can be deployed with an opened access opening or the access opening can be opened following deployment by inserting a needle or device to force open the hook or loop connectors 182, 184. Alternatively, the hook and loop connectors 182, 184 can be opened or separated by an opening tool (not shown) to form the access opening for treatment. Although a hook and loop interlockable structure is disclosed, other interlockable components can be used to form an openable/closable opening through the patch to access a body lumen or vessel for treatment and seal or close the body lumen or vessel following treatment.

FIGS. 8A-8D illustrate an alternate embodiment of a closure assembly which is deployable prior to incision to seal or close the incision following treatment. FIG. 8A shows that the closure material or seal includes a flowable media 200 which is disposed in a delivery sheath 116 that is used to deploy the flowable media proximate to an entry site into the lumen or cavity. As shown, the media 200 is deployed via a plunger or catheter 202 which is slidably disposed in the delivery sheath 116. The media 200 is formed of a material which sets such as a flowable gel or composition to form a sealing mass or body.

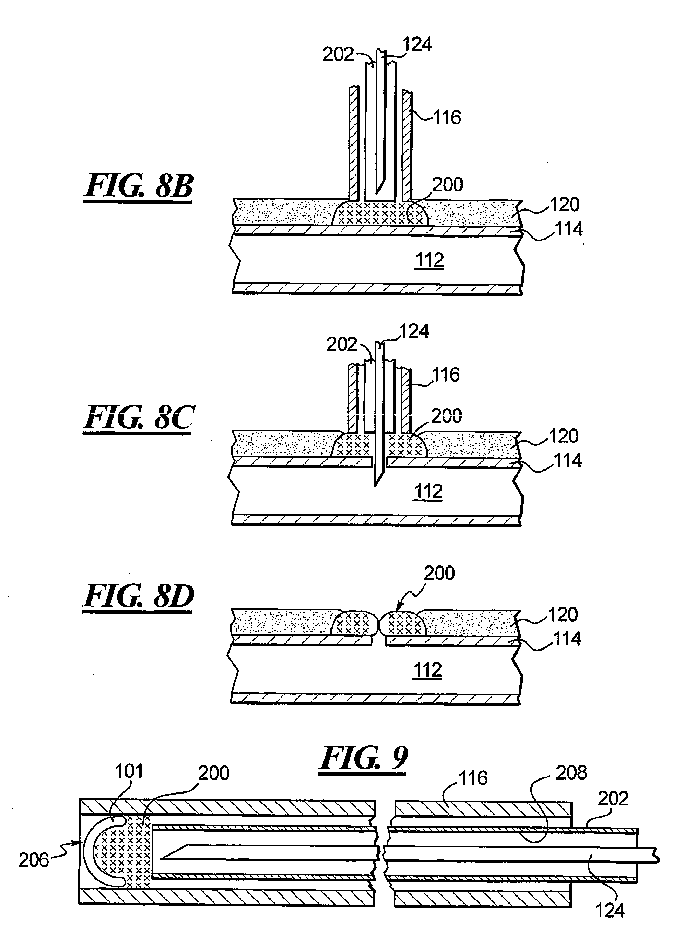

FIG. 8B shows that to deploy media 200, the delivery sheath 116 is inserted through the subcutaneous tissue 120. The plunger 202 is then advanced distally to position the media 200 proximate to the cavity or vessel wall. The media 200 may be allowed to somewhat solidify or set. Thereafter, or before the media 200 has set, needle 124 is advanced to puncture through the media 200 and form an incision through the vessel wall to access the lumen 112 for treatment as illustrated in FIG. 8C.

As previously described, catheters or treatment devices can be inserted through the media 200 and incision into the vessel lumen 112. For example, as previously described, catheters can be advanced through an introducer sheath which, for example, is inserted or tracked over the needle 124 into the lumen. Following treatment and withdrawal of the treatment devices or catheters, the media 200 provides a self sealing body which forms a seal or closure for the incision. In particular, the media 200 is formed of a self sealing material such as a clottable material, which provides a closure or seal as illustrated in FIG. 8D. For example, in one embodiment, a procoagulant, such as a flowable mixture of thrombin, collagen and diluent can be added to a gel to provide a puncturable media seal or closure.

In an embodiment illustrated in FIG. 9, the closure device or system includes a patch 101 and media 200 disposed in a delivery sheath 116 for deployment proximate to an entry site. As shown, the patch 101 is disposed proximate to a distal end of the delivery sheath 116 relative to the media 200 which is proximally located relative to patch 101. The system includes plunger 202 which is slidable along the delivery sheath 116 to expel the patch 101 and media 200 through a distal opening 206 of the delivery sheath 116. The media 200 is disposed between the patch 101 and plunger 202 so that movement of the plunger 202 distally expels or deploys the patch 101 first and media 200 second. This positions the combination of closure materials at the entry site prior to treatment.

Following deployment of the patch 101 and media 200, needle 124 is advanced to create an incision or opening in the vessel wall as described with respect to previous embodiments. The advancing needle 124 is initially forced through the media 200 and punctures (or is inserted through a pre-formed opening in) the patch 101 before reaching the vessel wall. The needle 124 is further advanced to create the incision in the vessel wall through the patch 101 and media 200-not shown in FIG. 9. In the illustrated embodiment, the needle 124 is slidably advanced through a lumen 208 of the plunger 202. In an alternate embodiment, the plunger 202 is withdrawn and the needle 124 is advanced either through the delivery sheath 116 or independent of the delivery sheath 116 to form the incision following deployment of the patch 101 and media 200.

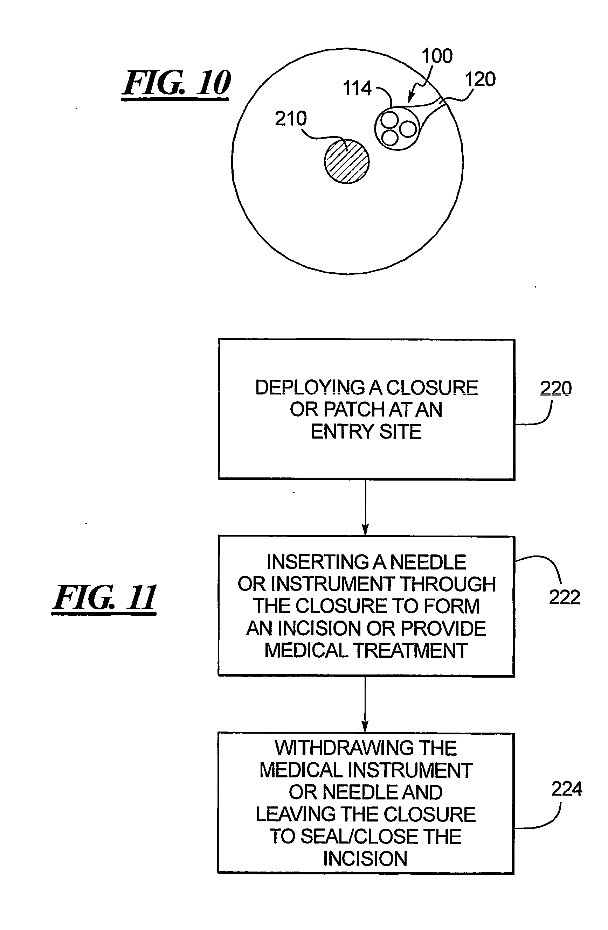

The closure system or device as described has a wide variety of applications. One particular application is for sealing an incision to a femoral artery following completion of a cardiac or percutaneous transluminal angioplasty procedure, and this is shown, by way of example, in FIG. 10. As described, the closure 100 (for example the patch 101 and/or media 200) are delivered through the subcutaneous tissue 120 at the femur 210 and deployed proximate to the vessel wall 114. Thus as described the closure is deployed pre-treatment or pre-insertion to seal an incision following treatment.

FIG. 11 is a block diagram illustrating steps for closing an incision following treatment via a closure device deployed prior to access or treatment. As illustrated by block 220, the patch or closure device is deployed at the entry site. Thereafter, as illustrated by block 222, a needle or instrument is inserted through the closure device either by puncturing the closure device or inserting the needle or instrument through a pre-formed opening in the closure device to form an incision in a body to access a body vessel or lumen, and treatment is performed. Following treatment, the needle and medical instruments are withdrawn leaving the closure device to seal or close the incision as illustrated by block 224. The method and steps disclosed does not form part of the invention but represents background art that is useful for understanding the invention.

## Claims

1. A medical device for accessing a body cavity or lumen, the device comprising:
a delivery sheath (116) having a distal end;
a needle (124);
a closure patch (101) disposed within the delivery sheath (116) and deployable from the distal end of the delivery sheath (116), the closure patch (101) comprising an opening (104, 105) for receiving the needle (124) therethrough or being puncturable (124) by the needle inserted therethrough, and
the needle (124) slidably disposed within the delivery sheath (116) with the patch (101) initially disposed between the needle (124) and the distal end of the sheath (116), the needle (124) being distally advancable through the patch (101) and out the distal end of the sheath (116) to form an incision through a body and into the body cavity through which a treatment device may be subsequently inserted;

2. The medical device of claim 1 wherein the patch (101) comprises a bioresorbable material.

3. The medical device of claim 1 wherein the patch (101) comprises an adhesive or adhesive layer (142),

4. The medical device of claim 1 wherein the patch (101) is self sealing to close the opening (104, 105) upon removal of the treatment device.

5. The medical device of claim 4 wherein the patch (101) comprises a self sealing gel material.

6. The medical device of claim 4 wherein the patch (101) comprises a clot forming material or coagulant material.

7. The medical device of claim 1 wherein the opening (104, 105) in the patch (101) is heat closable.

8. The medical device of claim 1 wherein the patch (101) comprises a heating element (150).

9. The medical device of claim 8 wherein the patch (101) comprises a heat sealable material (162) proximate to the opening (104, 105) and wherein the heating element (150) is configured to heat the heat sealable material (162) to close the opening (104, 105).

10. The medical device of claim 1 wherein the patch (101) comprises a pre-formed opening (104, 105) and a closure mechanism for closing the opening (104, 105) selected from the group consisting of a shape memory alloy (166), a super-elastic Nitinol closure, an electro-active polymer (168), bucky paper, a micro electromechanical closure (169) or combinations thereof.

11. The medical device of claim 1 wherein the opening (104, 105) is defined by a protruding body along a first portion of the opening (104, 105) insertable into a recess defined along a second portion of the opening (104, 105), such that insertion of the protruding body into the recess closes at least.a portion of the opening (104, 105).

12. The medical device of claim 1 wherein the patch (101) comprises a plurality of hook and loop fasteners (182, 184) positioned around the opening (104, 105) which is closable by the plurality of hook and loop fasteners (182, 184).

13. The medical device of claim 1 further comprising a flowable media (200) disposed within the sheath (116) proximal to the patch (101) prior to deployment.

## Patentansprüche

1. Medizinische Vorrichtung für den Zugang zu einem Körperhohlraum oder -lumen, wobei die Vorrichtung umfaßt:
eine Abgabehülle (116) mit einem distalen Ende;
eine Nadel (124);
ein Verschlußpflaster (101), das in der Abgabehülle (116) angeordnet ist und von dem distalen Ende der Abgabehülle (116) einsetzbar ist, wobei das Verschlußpflaster (101) eine Öffnung (104, 105) zur Aufnahme der dort hindurchgehenden Nadel (124) umfaßt oder von der hindurchgehend eingesteckten Nadel (124) durchstoßbar ist, und
wobei die Nadel (124) in der Abgabehülle (116) verschiebbar angeordnet ist, wobei das Pflaster (101) zunächst zwischen der Nadel (124) und dem distalen Ende der Hülle (116) angeordnet ist und wobei die Nadel (124) distal durch das Pflaster (101) und aus dem distalen Ende der Hülle (116) vorschiebbar ist, um einen Einschnitt, durch welchen eine Behandlungsvorrichtung nachfolgend eingeführt werden kann, in einem Körper und zu einem Körperhohlraum zu bilden.

2. Medizinische Vorrichtung nach Anspruch 1, wobei das Pflaster (101) ein bioresorbierbares Material aufweist.

3. Medizinische Vorrichtung nach Anspruch 1, wobei das Pflaster (101) einen Klebstoff oder eine Klebschicht (142) umfaßt.

4. Medizinische Vorrichtung nach Anspruch 1, wobei das Pflaster (101) selbstabdichtend ist, um die Öffnung (104, 105) nach Entfernung der Behandlungsvorrichtung zu verschließen.

5. Medizinische Vorrichtung nach Anspruch 4, wobei das Pflaster (101) ein selbstabdichtendes Gelmaterial umfaßt.

6. Medizinische Vorrichtung nach Anspruch 4, wobei das Pflaster (101) einen gerinselbildenden Stoff oder einen gerinnungsfördernden Stoff umfaßt.

7. Medizinische Vorrichtung nach Anspruch 1, wobei die Öffnung (104, 105) in dem Pflaster (101) durch Wärme verschließbar ist.

8. Medizinische Vorrichtung nach Anspruch 1, wobei das Pflaster (101) ein Heizelement (150) umfaßt.

9. Medizinische Vorrichtung nach Anspruch 8, wobei das Pflaster (101) ein durch Wärme verschließbares Material (162) in der Nähe der Öffnung (104, 105) umfaßt und wobei das Heizelement (150) dazu ausgebildet ist, das durch Wärme verschließbare Material (162) zu erwärmen, um die Öffnung (104, 105) zu schließen.

10. Medizinische Vorrichtung nach Anspruch 1, wobei das Pflaster (101) eine vorgeformte Öffnung (104, 105) und einen aus der aus einer Legierung mit Formgedächtnis (166), einem superelastischen Nitinol-Verschluß, einem elektroaktiven Polymer (168), Bucky Paper, einem mikroelektromechanischen Verschluß (169) oder einer Kombination davon bestehenden Gruppe ausgewählten Schließmechanismus zum Schließen der Öffnung (104, 105) umfaßt.

11. Medizinische Vorrichtung nach Anspruch 1, wobei die Öffnung (104, 105) durch einen entlang eines ersten Abschnitts der Öffnung (104, 105), der in eine entlang eines zweiten Abschnitts der Öffnung (104, 105) definierte Aussparung einsteckbar ist, herausragenden Körper definiert ist, so daß das Einstecken des herausragenden Körpers in die Aussparung mindestens einen Abschnitt der Öffnung (104, 105) schließt.

12. Medizinische Vorrichtung nach Anspruch 1, wobei das Pflaster (101) eine Mehrzahl von Klettverschlüssen (182, 184) umfaßt, welche um die durch die Mehrzahl von Klettverschlüssen (182, 184) verschließbare Öffnung (104, 105) angeordnet ist.

13. Medizinische Vorrichtung nach Anspruch 1, ferner umfassend ein vor dem Einsetzen innerhalb der Hülle (116) proximal des Pflasters (101) angeordnetes fließfähiges Medium (200).

## Revendications

1. Dispositif médical pour accéder à une cavité ou lumière corporelle, le dispositif comprenant :
une gaine de délivrance (116) ayant une extrémité distale ;
une aiguille (124) ;
une pastille de fermeture (101) disposée dans la gaine de délivrance (116) et déployable depuis l'extrémité distale de la gaine de délivrance (116), la pastille de fermeture (101) comprenant une ouverture (104, 105) pour recevoir l'aiguille (124) dans celle-ci ou étant perforable par l'aiguille (124) insérée dans celle-ci, et
l'aiguille (124) disposée à coulissement dans la gaine de délivrance (116) avec la pastille (101) disposée initialement entre l'aiguille (124) et l'extrémité distale de la gaine (116), l'aiguille (124) pouvant être amenée à avancer distalement à travers la pastille (101) et hors de l'extrémité distale de la gaine (116) pour former dans un corps et dans la cavité corporelle une incision par laquelle un dispositif de traitement peut ensuite être inséré.

2. Dispositif médical selon la revendication 1 où la pastille (101) comprend un matériau biorésorbable.

3. Dispositif médical selon la revendication 1 où la pastille (101) comprend un adhésif ou une couche adhésive (142).

4. Dispositif médical selon la revendication 1 où la pastille (101) est auto-étanchéifiante pour fermer l'ouverture (104, 105) lors du retrait du dispositif de traitement.

5. Dispositif médical selon la revendication 4 où la pastille (101) comprend un matériau de type gel auto-étanchéifiant.

6. Dispositif médical selon la revendication 4 où la pastille (101) comprend un matériau formant des caillots ou un matériau coagulant.

7. Dispositif médical selon la revendication 1 où l'ouverture (104, 105) dans la pastille (101) peut être fermée thermiquement.

8. Dispositif médical selon la revendication 1 où la pastille (101) comprend un élément chauffant (150).

9. Dispositif médical selon la revendication 8 où la pastille (101) comprend un matériau thermosoudable (162) à proximité de l'ouverture (104, 105) et où l'élément chauffant (150) est configuré pour chauffer le matériau thermosoudable (162) pour fermer l'ouverture (104, 105).

10. Dispositif médical selon la revendication 1 où la pastille (101) comprend une ouverture préformée (104, 105) et un mécanisme de fermeture pour fermer l'ouverture (104, 105) choisi dans le groupe consistant en un alliage à mémoire de forme (166), une fermeture Nitinol superélastique, un polymère électro-actif (168), un papier bombé, une fermeture micro-électromécanique (169) ou des combinaisons de ceux-ci.

11. Dispositif médical selon la revendication 1 où l'ouverture (104, 105) est définie par un corps en saillie le long d'une première partie de l'ouverture (104, 105) insérable dans un évidement défini le long d'une seconde partie de l'ouverture (104, 105) de sorte que l'insertion du corps en saillie dans l'évidement ferme au moins une partie de l'ouverture (104, 105).

12. Dispositif médical selon la revendication 1 où la pastille (101) comprend une pluralité d'éléments de fixation à crochets et boucles (182, 184) positionnés autour de l'ouverture (104, 105) qui peut être fermée par la pluralité d'éléments de fixation à crochets et boucles (182, 184).

13. Dispositif médical selon la revendication 1 comprenant en outre un milieu fluide (200) disposé dans la gaine (116) à proximité de la pastille (101) avant le déploiement.
